# EUROPEAN PATENT APPLICATION

(11) **EP 1 854 492 A2**
(43) Date of publication of application: **14.11.2007**
(21) Application number: 07251964.8
(22) Date of filing: 11.05.2007
(51) Int. Cl.: A61M 1/16, A61J 1/05

(54) **Reservoir assembly for container holding bicarbonate solution**

(30) Priority: 12.05.2006 JP 2006133641
(71) Applicant: Ajinomoto Co., Inc., Tokyo 104-8315 (JP)
(72) Inventor: Kitagawa, Shouichi, Kawasaki-shi Kanagawa 210-8681 (JP); Shigeta, Mutsuo, Kawasaki-shi Kanagawa 210-8681 (JP)
(74) Representative: Nicholls, Kathryn Margaret

(57) **Abstract**

A reservoir assembly can stably hold a bicarbonate solution. The reservoir assembly includes a gas-permeable plastic container holding a bicarbonate solution and a gas-impermeable casing for encasing the container. An effective way of detecting pinholes in the casing of the reservoir assembly is also provided. An oxygen absorber and an oxygen-detecting agent are placed in the space between the container and the casing. The oxygen absorber is of the type that does not affect the pH or the bicarbonate ion concentration of the solution and mainly contains a crosslinked polymer having carbon-carbon unsaturated bonds.

## Description

The present invention relates to a reservoir assembly for accommodating a plastic container holding a bicarbonate solution. In particular, the present invention relates to a reservoir assembly comprising a gas-impermeable casing for encasing the plastic container. Furthermore, the present invention relates to detecting pinholes in the casing of the reservoir assembly. The reservoir assembly of the present invention can effectively be used for stable preservation of such a bicarbonate solution.

Major purposes of traditional renal replacement therapies, including hemodialysis, hemodiafiltration and peritoneal dialysis, include not only removal of water and metabolites, but also correction of metabolic acidosis. For example, hemodialysis, the most common blood purification technique used to treat patients with chronic renal failure (CRF), is intended to correct the serum electrolyte levels and acid-base balance, as well as to remove waste' products and water from the blood.

Dialysates used in hemodialysis must contain significant amounts of alkalizers in order to correct the metabolic acidosis caused by the loss of bicarbonate ions in patients with CRF. The most suitable alkalizer therefore is bicarbonate itself and, thus, most dialysates for use in hemodialysis contain bicarbonates. However, bicarbonate dialysates are less stable since bicarbonate ions present in these dialysates tend to react with calcium ions and magnesium ions, forming insoluble compounds (i.e., metal carbonates such as calcium carbonate and magnesium carbonate). Also, bicarbonate dialysates are susceptible to bacterial growth and are unsuitable for long-term storage.

One solution to these problems is to use acetic acid. Acetic acid is known to be metabolized, and is converted to bicarbonate in liver. Thus, by using an acetate, such as sodium acetate, as an alkalizer, stable dialysates can be obtained that can effectively replace alkali. Although acetate dialysates can contain alkalis at higher concentrations and ensure sufficient supply of bicarbonate ions, acetic acid acts to dilate blood vessels or suppress cardiac functions, causing a low blood pressure. In addition to this, when acetate dialysates are used in patients with acetate intolerance, a condition characterized by slow acetic acid metabolism, acetic acid causes aggravation of dialysis disequilibrium syndrome or respiratory depression resulting from the significant loss of blood carbon dioxide during dialysis.

As the number of dialysis patients increases, as does the number of patients who have diabetic nephropathy as primary disease, the frequency and severity of dialysis disequilibrium syndrome are on the increase. Furthermore, there is an increasing demand among dialysis patients for symptom-free dialysis that poses little burden on patients. In addition, involvement of acetic acid in these symptoms is suspected. For these reasons, acetate dialysis that uses acetate as alkalizer has been replaced by bicarbonate dialysis that uses sodium bicarbonate as alkalizer.

The bicarbonate solution (dialysate) used in bicarbonate dialysis is generally provided as an aqueous solution containing sodium bicarbonate. When desired, the aqueous solution may further contain electrolytes or sugars. Sodium bicarbonate in the bicarbonate solution tends to decompose into sodium carbonate and carbon dioxide. As the reaction progresses, the release of carbon dioxide and the production of sodium carbonate result in an increase in the pH of the solution over time. When the bicarbonate solution with an increased pH is administered to patients, it cannot achieve its intended purpose: to properly correct the metabolic acidosis. Rather, the bicarbonate solution with an increased pH can cause side effects.

When the bicarbonate solution is packaged in a gas-impermeable glass container, the pH of the solution remains stable since the generated carbon dioxide is not released from the reaction system and is reabsorbed by the solution. However, glass containers are not readily handleable: They are heavy, can break easily, and are difficult to dispose of after use. Thus, glass containers have been replaced by plastic containers in recent years.

Plastic containers used for this purpose are generally made of a highly gas-permeable material. When the bicarbonate solution is packaged in such a gas-permeable plastic container, the carbon dioxide produced during heat sterilization or during long-term storage is not reabsorbed by the solution and is released from the container. As a result, the pH of the bicarbonate solution varies.

For this reason, the container holding the bicarbonate solution is typically encased in a gas-impermeable casing to prevent changes in the solution caused by the release of carbon dioxide.

However, when a pinhole is formed in the casing, carbon dioxide released from the plastic container is further released outside the casing. This allows further decomposition of bicarbonate ions and resulting increase in the pH. Should the pH increase, the alkaline preparation (solution) can cause alkalosis when administered to patients. In a one solution-type preparation in which bicarbonate ions are formulated with metals such as calcium and/or magnesium to form a single formulation, the increased pH can result in crystallization of insoluble carbonates, such as calcium carbonate.

Any pinhole formed in the casing holding the bicarbonate solution can significantly affect the safety and stability of the preparation and some countermeasures must be taken to detect such pinholes (See, for example, Patent Document 1).

One approach to detect pinholes is to use oxygen-detecting agents that detect oxygen flowing into the casing from the ambient air. Such oxygen-detecting agents are currently in practical use. However, to use these oxygen-detecting agents, the oxygen concentration inside the casing must be maintained at 0.1% or below. This requires the use of an oxygen absorber (See, for example, Patent Document 2).

Oxygen absorbers based on iron powder are commonly known and are used for this purpose. However, active carbon and iron powder used in these iron powder-based oxygen absorbers absorb not only oxygen, but also carbon dioxide, making the oxygen absorbers unsuitable for use in bicarbonate preparations. Thus, two alternative approaches are employed to improve stability of bicarbonate solutions. In one approach, the space between the container holding the bicarbonate solution and the casing is filled with carbon dioxide of a particular concentration (See, for example, Patent Document 3). In the other approach, carbon dioxide-releasable oxygen absorbers that release carbon dioxide upon oxygen absorption are used (See, for example, Patent Document 4).

In the first approach, care must be taken to maintain the concentration of carbon dioxide to fill the space between the container holding the bicarbonate solution and the casing within a predetermined range. Too high a concentration of carbon dioxide would lead to a decrease in the pH of the bicarbonate solution and to an increase in the amount of bicarbonate in the solution. The second approach also has a problem that the quality of the resulting products varies significantly. The reason for this is as follows: While carbon dioxide generated by the carbon dioxide-releasable oxygen absorber quickly reaches equilibrium with bicarbonate ions in the bicarbonate solution and stabilizes the solution, the amount of carbon dioxide generated tends to vary depending on the amount of residual oxygen present in the preparation (either in the bicarbonate solution, in the space inside the container, or in the casing) immediately after the production.

Since the amount of carbon dioxide required to stabilize the bicarbonate solution varies depending also on the pH of the solution and the concentration of bicarbonate ions, the generation of carbon dioxide in small amounts can have non-negligible effects on the quality of each preparation. Thus, the use of oxygen absorbers that produce little carbon dioxide has been proposed (See, for example, Patent Document 5). In this approach, the concentration of carbon dioxide in the space between the container holding the bicarbonate solution and the casing is not particularly adjusted and the oxygen absorber that produces little oxygen is used to stabilize the solution. It has turned out, however, that the pH of the bicarbonate solution changes during storage.
Patent Document 1 Japanese Patent Laid-Open Publication No. Hei 11-139561
Patent Document 2 Japanese Patent Laid-Open Publication No. Hei 8-164185
Patent Document 3 Japanese Patent No. 2750373
Patent Document 4 Japanese Patent No. 2505329
Patent Document 5 Japanese Patent Laid-Open Publication No. 2001-192069

Accordingly, it is an object of the present invention to provide a reservoir assembly for a container holding a bicarbonate solution, comprising a gas impermeable casing for encasing a plastic container holding a bicarbonate solution. It is another object of the present invention to provide an effective way of detecting pinholes in the casing of the reservoir assembly. The reservoir assembly of the present invention can effectively be used for stable preservation of such a bicarbonate solution.

In the course of our study to achieve these objects, the present inventors hypothesized that with the general stability of bicarbonate solution being ensured by the gas barrier property of the casing of the solution container, the effect on the pH and the bicarbonate ion concentration of the bicarbonate solution can be substantially eliminated by preventing changes in the concentration of carbon dioxide in the space between the solution container and the casing for encasing the container.

Based on this hypothesis, the present inventors have demonstrated that an accurate pinhole detection system can be established by adjusting the concentration of carbon dioxide in the space between the solution container and the casing in such a manner that the concentration of carbon dioxide in the headspace of the solution container is maintained in equilibrium with the pH of the solution, and by placing in that space an oxygen absorber that serves to absorb oxygen, but does not produce or absorb carbon dioxide. Such a pinhole detection system can stably detect any pinhole in the casing independent of the volume of solution, concentration of bicarbonate ions, residual oxygen in the casing or other factors. The present inventors have thus completed the invention.

Thus, one essential aspect of the present invention concerns a reservoir assembly. The invention of claim 1, one embodiment of the first aspect, is the reservoir assembly, comprising a gas-permeable plastic container holding a bicarbonate solution, a gas-impermeable casing encasing the container, and an oxygen absorber and an oxygen-detecting agent placed in a space between the container and the casing, wherein the oxygen absorber does not affect the pH or the bicarbonate ion concentration of the solution.

A more specific embodiment of the first aspect, the invention of claim 2 is the reservoir assembly according to claim 1, wherein the oxygen absorber placed in the space between the container and the casing mainly contains a crosslinked polymer having carbon-carbon unsaturated bonds.

The oxygen absorber serves to absorb oxygen, but does not produce or absorb carbon dioxide.

A more specific embodiment of the first aspect, the invention of claim 3 is the reservoir assembly according to claim 1 or 2, wherein the space between the container and the casing is filled with a gas atmosphere having a carbon dioxide concentration in equilibrium with the pH of the solution, so that changes in the pH of the solution and in the amount of carbon dioxide in the container are minimized during packaging of the container.

A more specific embodiment of the first aspect, the invention of claim 4 is the above-described reservoir assembly, wherein the gas-impermeable casing has oxygen permeability of 0.7 mL/m²/day/atom or less.

The invention of claim 5 is the above-described reservoir assembly, wherein the solution has a pH in the range of 6.8 to 7.8. The invention of claim 6 is the above-described reservoir assembly, wherein the gas atmosphere filling the space between the container and the casing contains carbon dioxide at a concentration in the range of 1 to 19 v/v% and oxygen at a concentration of 0.1% or less.

The reservoir assembly of the present invention, comprising a container holding a bicarbonate solution and a casing for encasing the solution container, is preferably configured such that the concentration of carbon dioxide in the space between the container and the casing is adjusted in such a manner that the concentration of carbon dioxide in the headspace of the container is maintained in equilibrium with the pH of the solution, and in that an oxygen absorber that does not affect the pH or the bicarbonate ion concentration of the solution is placed in that space along with an oxygen-detecting agent.

Such characteristics of embodiments of the invention prevent changes in the carbon dioxide concentration in the headspace of the container or in the space between the container and the casing. As a result, the pH of the bicarbonate solution in the reservoir assembly is stabilized.

Accordingly, embodiments of the present invention may provide a stable, side effect-free bicarbonate solution that does not change its bicarbonate ion concentration.

The reservoir assembly of the present invention is manufactured by filling of a container with a bicarbonate solution and packaging the container in a casing. The use of the oxygen absorber that does not affect the pH or the bicarbonate ion concentration of the solution helps prevent changes in the pH of the solution during packaging of the container. In addition, the pH of the solution remains stable independent of the amount of residual oxygen.

Bicarbonate solution for use in the present invention is a solution containing sodium bicarbonate to serve as an alkalizer desirably in an amount of 20 to 35 mEq/L, and preferably in an amount of 22 to 30 mEq/L. Preferably, the bicarbonate solution further contains electrolytes, such as 130 to 145 mEq/L of sodium ion, 2 to 5 mEq/L of potassium ion, 90 to 130 mEq/L of chloride ion, 2 to 5 mEq/L of calcium ion, 0.5 to 2.5 mEq/L of magnesium ion, 1 to 7 mEq/L of citrate ion, and 0 to 5 g/L of glucose.

Any of these electrolytes may be used as desired. Specific examples include sodium chloride, sodium citrate, sodium acetate, sodium lactate, sodium dihydrogen phosphate, disodium hydrogen phosphate, sodium gluconate, sodium glycerophosphate, sodium malate, potassium chloride, dipotassium phosphate, potassium acetate, potassium citrate, potassium lactate, potassium glycerophosphate, potassium malate, calcium chloride, calcium lactate, calcium gluconate, calcium glycerophosphate, dibasic calcium phosphate, calcium malate, magnesium chloride, magnesium gluconate and calcium glycerophosphate.

A particularly preferred bicarbonate solution contains sodium chloride, potassium chloride, calcium chloride, magnesium chloride, sodium bicarbonate, sodium citrate and/or glucose.

Sodium bicarbonate used in bicarbonate solutions tends to react with calcium and magnesium to form insoluble calcium carbonate and magnesium carbonate. Furthermore, when left to stand or heated, an aqueous sodium bicarbonate solution releases carbon dioxide, resulting in an increase in the pH of the solution. It has thus been considered difficult to obtain stable solutions and these solutions are formulated either upon use or as a two solution-type preparation composed of a sodium bicarbonate solution and an electrolyte solution. The two solutions may be packaged in two separate containers.

According to the present invention, bicarbonate solutions can be formulated as a single solution for the following reasons: The container holding the solution is encased in a gas-impermeable casing and the carbon dioxide concentration is kept in equilibrium with the pH of the solution in the headspace of the container as well as in the space between the container and the casing; and the oxygen absorber that does not affect the pH or the bicarbonate ion concentration of the solution is placed in the space along with the oxygen-detecting agent.

The bicarbonate solutions may of course be provided in separate containers or as a two solution-type preparation.

The container for holding the bicarbonate solution may be any pharmaceutically acceptable plastic container that passes gases, but not liquids. Examples of such plastic containers include containers made of thermoplastic resin films of polyethylene, polypropylene or polyvinylchloride.

In the reservoir assembly of the present invention, the gas-impermeable casing for encasing the gas-permeable plastic container is preferably made of thermoplastic material that is particularly impermeable to carbon dioxide. Examples of such thermoplastic materials include ethylene-vinyl alcohol copolymer (EVOH), poly(vinylidene chloride) (PVDC) and polyethylene terephthalate (PET), and films on which aluminum oxide or silicon oxide has been deposited by vapor deposition. Laminated films made of aluminum foils may also be used.

In the production of the reservoir assembly of the present invention, an oxygen-detection agent and an oxygen absorber are placed in the space formed between the container holding bicarbonate solution and the casing for encasing the container during the packaging of the container in the casing. The oxygen absorber is preferably arranged so that some space remains around the oxygen absorber to facilitate oxygen absorption by the agent.

The oxygen-detecting agent for use in the present invention may be any agent that changes its physical properties in the presence of oxygen. One example is AGELESS EYE manufactured by Mitsubishi Gas Chemical Co., Ltd.

The oxygen absorber for use in the present invention affects neither the pH nor the bicarbonate ion concentration of the solution. In other words, the oxygen absorber serves only to absorb oxygen: It does not have additional functions, such as generation and absorption of carbon dioxide.

Examples of such oxygen absorbers include those that mainly contain a crosslinked polymer having carbon-carbon unsaturated bonds. Examples include an oxygen absorber described in Japanese Patent Laid-Open Publication No. Hei 11-347400.

The correlation between the pH and the carbon dioxide concentration of the solution can be determined by measuring the equilibrium carbon dioxide concentration in the headspace of the container holding a solution with altered pH.

Theoretically, there is a substantially linear relationship between the pH and the carbon dioxide concentration in a solution at equilibrium. Even when the pH and the carbon dioxide concentration are within the respective ranges specified above, it is more preferred that the pH falls within the range of 6.8 to 7.1 and the carbon dioxide concentration within the range of 9 to 19 v/v% to ensure equilibrium with the carbon dioxide concentration.

An exemplary process for the production of the reservoir assembly of the present invention is now described.

First, a bicarbonate solution is prepared that contains 20 to 35 mEq/L of sodium bicarbonate as an alkalizer and electrolytes. 100% carbon dioxide gas is bubbled through the solution to decrease the pH of the solution to approximately 6.6.

Next, the solution is filtered and a desired amount is packaged in a gas-impermeable plastic container. Alternatively, a desired amount of the solution may be sent into the container using pressurized nitrogen gas. This reduced the oxygen concentration in the headspace of the container.

At this stage, the pH of the solution slightly increases.

The filled solution container is sterilized by heating at about 105°C for 15 to 30 min. After heating, the bicarbonate solution has a desired pH value and is in equilibrium with the carbon dioxide concentration in the headspace of the container.

The container is then packaged in a gas-impermeable casing. An oxygen absorber that does not affect the pH or the bicarbonate ion concentration of the solution is then placed, along with an oxygen-detection agent, in the space between the container and the casing. A mixture of carbon dioxide gas and nitrogen gas is then blown into the casing. The concentration of carbon dioxide in the mixture is in equilibrium with the carbon dioxide concentration in the headspace of the container. The casing is then sealed to complete the reservoir assembly.

It is virtually impossible to completely eliminate the residual oxygen dissolved in the bicarbonate solution. Conventional reservoirs for containing bicarbonate solutions include an oxygen absorber placed in the space between the container and the casing encasing the container. As the oxygen absorber absorbs oxygen, the concentration of carbon dioxide in the space changes, which in turn causes a change in the pH of the solution.

Conversely, the reservoir assembly of the present invention prevents changes in the pH of the solution during packaging of the container by maintaining the carbon dioxide concentration in the headspace of the solution container and in the space between the container and the casing in equilibrium with the pH of the solution. In addition to this, the oxygen absorber used in the present invention is of the type that affects neither the pH nor the bicarbonate ion concentration of the solution, so that the pH of the solution will not be affected by the residual oxygen presents in the casing immediately after packaging of the solution container in the casing.

### Examples

Embodiments of the present invention will now be described in detail with reference to Examples.

### Example 1:

The components shown in Table 1 below were dissolved in a predetermined amount of distilled water at room temperature at concentrations given in the table to form a solution. Carbon dioxide was bubbled through the resulting solution until the pH was 6.5. Subsequently, the solution was filtered through a 0.22µm membrane filter. 500 ml of the filtrate was packaged in a polyethylene container and the container was sealed, leaving a headspace of approximately 50 ml. The sealed container was autoclaved at 105°C for 25 min.

Subsequently, the filled solution container was encased in a gas-impermeable secondary casing. An oxygen absorber (AGELESS GP, Mitsubishi Gas Chemical Co., Ltd.), mainly containing a crosslinked polymer having carbon-carbon unsaturated bonds, was then placed, along with an oxygen-detecting agent, in the space formed between the container and the casing. The air in that space was then replaced by nitrogen. This completed a reservoir assembly.

**Table 1**

| Components | Concentration (g/L) |
|---|---|
| Sodium chloride | 6.14 |
| Potassium chloride | 0.30 |
| Calcium chloride | 0.22 |
| Magnesium chloride | 0.102 |
| Sodium bicarbonate | 2.10 |
| Sodium citrate | 0.490 |

### Comparative Example 1:

A reservoir assembly of Comparative Example 1 was produced in the same manner as in Example 1, except that the oxygen absorber (AGELESS GP) was replaced by an iron powder-based oxygen absorber (AGELESS ZP, Mitsubishi Gas Chemical Co., Ltd.) capable of absorbing carbon dioxide.

### Comparative Example 2:

A reservoir assembly of Comparative Example 2 was produced in the same manner as in Example 1, except that neither the oxygen absorber nor the oxygen-detecting agent was used.

### Example 2:

A reservoir assembly of Example 2 was produced in the same manner as in Example 1, except that the pH of the solution was adjusted by carbon dioxide bubbling to a value of 6.8.

### Comparative Example 3:

A reservoir assembly of Comparative Example 3 was produced in the same manner as in Example 1, except that the pH of the solution was adjusted by carbon dioxide bubbling to a value of 6.8 and that the oxygen absorber (AGELESS GP) was replaced by an oxygen absorber (AGELESS GT, Mitsubishi Gas Chemical Co., Ltd.) capable of releasing carbon dioxide.

### Comparative Example 4:

A reservoir assembly of Comparative Example 4 was produced in the same manner as in Example 1, except that the pH of the solution was adjusted by carbon dioxide bubbling to a value of 6.8 and that neither the oxygen absorber nor the oxygen-detecting agent was used.

### Test Example 1: Changes in the pH of solution during long-term storage

Each of the reservoir assemblies of Examples 1 and 2 and Comparative Examples 1 through 4 was stored at 40°C for 6 months. The pH of each solution was measured 1, 2 and 6 months after the start of the storage period.

The results are shown in Table 2 below.

**Table 2**

| | Initial | Stored at 40° C | | |
|---|---|---|---|---|
| | | 1 M | 2 M | 6 M |
| Example 1 (AGELESS GP/replaced nitrogen) | 6.74 | 7.08 | 7.07 | 7.14 |
| Comparative Example 1 (AGELESS ZP/replaced nitrogen) | 6.74 | 7.26 | 7.26 | 7.51 |
| Comparative Example 2 (No oxygen absorber/replaced nitrogen) | 6.74 | 7.02 | 7.03 | 7.14 |
| Example 2 (AGELESS GP/replaced nitrogen) | 7.10 | 7.40 | 7.40 | 7.48 |
| Comparative Example 3 (AGELESS GT/replaced nitrogen) | 7.10 | 7.31 | 7.28 | 7.34 |
| Comparative Example 4 (No oxygen absorber/replaced nitrogen) | 7.10 | 7.31 | 7.39 | 7.49 |

### Example 3:

The components shown in Table 1 above were dissolved in a predetermined amount of distilled water at room temperature at concentrations given in the table to form a solution. Carbon dioxide was bubbled through the resulting solution until the pH was 6.6. Subsequently, the solution was filtered through a 0.22µm membrane filter. 500 ml of the filtrate was packaged in a polyethylene container. The air in the headspace of the container was replaced by nitrogen and the container was sealed. The sealed container was autoclaved at 105°C for 23 min.

Subsequently, the filled solution container was encased in a gas-impermeable secondary casing. An oxygen absorber (AGELESS GP) and an oxygen-detecting agent were then placed in the space formed between the container and the casing. The air in this space was then replaced by nitrogen. The resulting reservoir was packaged and stored at room temperature for 2 weeks. After the storage period, oxygen in the package was completely absorbed, the pH of the solution was 7.1, and the carbon dioxide concentration in the headspace was 10 v/v%.

The package was then opened and the gas in the headspace was replaced by 85 mL of a gaseous mixture of 18% oxygen, 10v/v% carbon dioxide and the remainder of nitrogen. The solution container was then encased in a gas-impermeable secondary casing. An oxygen absorber (AGELESS GP) and an oxygen-detecting agent were placed in the space formed between the container and the casing. The air in this space was replaced by 400 ml of a gaseous mixture of 5% oxygen, 10% carbon dioxide and the reminder of nitrogen. This completed a reservoir assembly of Example 3.

### Comparative Example 5:

A reservoir assembly of Comparative Example 5 was produced in the same manner as in Example 3, except that AGELESS GP placed upon the second packaging was replaced by AGELESS ZP.

### Comparative Example 6:

A reservoir assembly of Comparative Example 6 was produced in the same manner as in Example 3, except that AGELESS GP placed upon the second packaging was replaced by AGELESS GT.

### Comparative Example 7:

A reservoir assembly of Comparative Example 7 was produced in the same manner as in Example 3, except that AGELESS GP placed upon the second packaging was not used.

### Test Example 2: Changes in the pH of solution during storage

Each of the reservoir assemblies of Example 3 and Comparative Examples 5 through 7 was stored at 40°C for 2 weeks. Subsequently, the pH of each solution was measured, as well as the carbon dioxide and oxygen concentrations both in the space between the solution container and the casing and in the headspace of the solution container.

The results are shown in Table 3 below.

**Table 3**

| | Measured properties | | 40° C | |
|---|---|---|---|---|
| | | | 1 W | 2 W |
| Example 3 (AGELESS GP) | pH | | 7.11 | 7.09 |
| | Space | CO₂ Conc. | 9.7v/v% | 9.8v/v% |
| | | O₂ Conc. | 0.0% | 0.0% |
| | Headspace | CO₂ Conc. | 8.6v/v% | 9.2v/v% |
| | | O₂ Conc. | 1.1% | 0.1% |
| Comparative Example 5 (AGELESS ZP) | pH | | 7.40 | 7.51 |
| | Space | CO₂ Conc. | 5.4v/v% | 3.7v/v% |
| | | O₂ Conc. | 0.0% | 0.0% |
| | Headspace | CO₂ Conc. | 5.5v/v% | 3.8v/v% |
| | | O₂ Conc. | 1.3% | 0.1% |
| Comparative Example 6 (AGELESS GT) | pH | | 6.98 | 6.96 |
| | Space | CO₂ Conc. | 13.2v/v% | 12.9v/v% |
| | | O₂ Conc. | 0.0% | 0.0% |
| | Headspace | CO₂ Conc. | 9.7v/v% | 12.1v/v% |
| | | O₂ Conc. | 1.4% | 0.1% |
| Comparative Example 7 (No oxygen absorber) | pH | | 7.11 | 7.08 |
| | Space | CO₂ Conc. | 10.7v/v% | 10.2v/v% |
| | | O₂ Conc. | 7.1% | 7.1% |
| | Headspace | CO₂ Conc. | 9.6v/v% | 9.8v/v% |
| | | O₂ Conc. | 7.7% | 7.2% |

### Example 4:

The components shown in Table 1 above were dissolved in a predetermined amount of distilled water at room temperature at concentrations given in the table to form a solution. Carbon dioxide was bubbled through the resulting solution until the pH was 6.6. Subsequently, the solution was filtered through a 0.22µm membrane filter. 500 ml of the solution was packaged in a polyethylene container. The air in the headspace of the container was replaced by nitrogen and the container was sealed. The sealed container was autoclaved at 105°C for 25 min.

Subsequently, the filled solution container was encased in a gas-impermeable secondary casing. An oxygen absorber (AGELESS GP) and an oxygen-detecting agent were then placed in the space formed between the solution container and the casing. The air in that space was then replaced by a gaseous mixture of carbon dioxide and nitrogen with the carbon dioxide concentration being the same as the gas in the headspace of the solution container. The resulting reservoir was packaged to make a reservoir assembly of Example 4.

### Example 5:

A reservoir assembly of Example 5 was produced in the same manner as in Example 4, except that the pH of the solution was adjusted by carbon dioxide bubbling to a value of 6.5.

### Example 6:

A reservoir assembly of Example 6 was produced in the same manner as in Example 4, except that the pH of the solution was adjusted by carbon dioxide bubbling to a value of 6.6.

### Example 7:

A reservoir assembly of Example 7 was produced in the same manner as in Example 4, except that the pH of the solution was adjusted by carbon dioxide bubbling to a value of 6.7.

### Comparative Example 8:

A reservoir assembly of Comparative Example 8 was produced in the same manner as in Example 4, except that the air in the space between the solution container and the casing was evacuated for packaging without using the oxygen absorber (AGELESS GP) or the oxygen-detecting agent (vacuum packaging).

### Comparative Example 9:

A reservoir assembly of Comparative Example 9 was produced in the same manner as in Example 4, except that the pH of the solution was adjusted by carbon dioxide bubbling to a value of 6.5 and that the air in the space between the solution container and the casing was evacuated for packaging without using the oxygen absorber (AGELESS GP) or the oxygen-detecting agent (vacuum packaging).

### Comparative Example 10:

A reservoir assembly of Comparative Example 10 was produced in the same manner as in Example 4, except that the pH of the solution was adjusted by carbon dioxide bubbling to a value of 6.6 and that the air in the space between the solution container and the casing was evacuated for packaging without using the oxygen absorber (AGELESS GP) or the oxygen-detecting agent (vacuum packaging).

### Comparative Example 11:

A reservoir assembly of Comparative Example 11 was produced in the same manner as in Example 4, except that the pH of the solution was adjusted by carbon dioxide bubbling to a value of 6.7 and that the solution container was vacuum-packaged after encased in a gas-impermeable casing without using the oxygen absorber or the oxygen-detecting agent.

### Test Example 3: Changes in the pH of solution during storage

Each of the reservoir assemblies of Examples 4 through 7 and Comparative Examples 8 through 11 was stored at 25°C for 2 weeks. Subsequently, the pH of each solution was measured, as well as the carbon dioxide and oxygen concentrations in the headspace of the solution container.

The results are shown in Table 4 below.

**Table 4**

| | Measured Properties | Initial | 25°C | |
|---|---|---|---|---|
| | | | 1 W | 2 W |
| Example 4 | pH | 6.80 | 6.83 | 6.81 |
| (AGELESS GP/ Gas replacement packaging) | CO₂ Conc. | 18.2v/v% | 17.5v/v% | 17.5v/v% |
| | O₂ Conc. | N/A | 0.9% | 0.2% |
| Comparative Example 8 | pH | 6.80 | 6.85 | 6.82 |
| (No oxygen absorber/ Vacuum packaging) | CO₂ Conc. | 18.2v/v% | 16.7v/v% | 17.2v/v% |
| | O₂ Conc. | N/A | 4.9% | 4.7% |
| Example 5 | pH | 6.91 | 6.92 | 6.92 |
| (AGELESS GP/ Gas replacement packaging) | CO₂ Conc. | 14.2v/v% | 13.8v/v% | 13.5v/v% |
| | O₂ Conc. | N/A | 1.0% | 0.2% |
| Comparative Example 9 | pH | 6.91 | 6.93 | 6.92 |
| (No oxygen absorber/ Vacuum packaging) | CO₂ Conc. | 14.2v/v% | 13.3v/v% | 13.5v/v% |
| | O₂ Conc. | N/A | 5.2% | 5.1% |
| Example 6 | pH | 7.00 | 6.99 | 6.99 |
| (AGELESS GP/ Gas replacement packaging) | CO₂ Conc. | 11.9v/v% | 11.7v/v% | 11.6v/v% |
| | O₂ Conc. | N/A | 0.9% | 0.2% |
| Comparative Example 10 | pH | 7.00 | 7.02 | 7.02 |
| (No oxygen absorber/ Vacuum packaging) | CO₂ Conc. | 11.9v/v% | 11.1v/v% | 10.9v/v% |
| | O₂ Conc. | N/A | 5.1% | 5.5% |
| Example 7 | pH | 7.09 | 7.09 | 7.09 |
| (AGELESS GP/ Gas replacement packaging) | CO₂ Conc. | 9.6v/v% | 9.4v/v% | 9.4v/v% |
| | O₂ Conc. | N/A | 0.9% | 0.2% |
| Comparative Example 11 | pH | 7.09 | 7.11 | 7.10 |
| (No oxygen absorber/ Vacuum packaging) | CO₂ Conc. | 9.6v/v% | 9.4v/v% | 8.9v/v% |
| | O₂ Conc. | N/A | 5.6% | 5.5% |

The results of Examples, Comparative Examples and Test Examples demonstrate the following.

The results of Test Example 1 for the stability during long-term storage show that the solution in each of the reservoir assemblies of Examples and Comparative Examples released carbon dioxide inside the reservoir assembly, increasing the pH of each solution immediately after the start of the storage period. However, the changes in the pH observed for Examples (with AGELESS GP) after one month or later significantly deviated from those observed for Comparative Examples using other types of oxygen absorber, but were substantially the same as those observed for oxygen absorber-free Comparative Examples. This indicates that unlike other oxygen absorbers, AGELESS GP does not cause pH changes due to its inability to absorb or release carbon dioxide. Thus, the long-term stability of solution is ensured by the use of AGELESS GP.

In Test Example 2 for storage stability, certain amounts of oxygen were present inside each reservoir assembly immediately after packaging. It has been proven that unlike other oxygen absorbers, the use of Ageless GP, which does not absorb or release carbon dioxide while it absorbs oxygen, enabled oxygen removal from within the reservoir assembly without causing significant pH changes.

AGELESS ZP, which absorbs carbon dioxide along with oxygen, resulted in an increased pH, whereas AGELESS GT, which releases carbon dioxide, decreased the pH.

In Test Example 3 for storage stability, solutions having different pH values were packaged with AGELESS GP. The results indicate that no significant pH changes were observed in each Example during the storage period. It has thus been demonstrated that AGELESS GP can be packaged with a gaseous mixture having a carbon oxide concentration corresponding to the pH of the solution so as to effectively remove oxygen from within the reservoir assembly without causing significant pH changes.

As set forth, the present invention provides a reservoir assembly comprising a container holding bicarbonate solution and a gas-impermeable casing for encasing the solution container. The concentration of carbon dioxide in the space between the container and the casing is adjusted in such a manner that the concentration of carbon dioxide in the headspace of the solution container is maintained in equilibrium with the pH of the solution. An oxygen absorber that does not affect the pH or the bicarbonate ion concentration of the solution is placed in the space along with an oxygen-detecting agent.

Thus, significant changes in the carbon dioxide concentration can be avoided in the headspace of the solution container, as well as in the space between the solution container and the casing. As a result, the pH of the bicarbonate solution in the reservoir assembly is stabilized. The present invention is of significant medical importance in that it provides stable, side effect-free bicarbonate solutions that do not change bicarbonate ion concentration.

## Claims

1. A reservoir assembly for a container holding a bicarbonate solution, comprising:
a gas-permeable plastic container holding a bicarbonate solution;
a gas-impermeable casing encasing the container; and
an oxygen absorber and an oxygen-detecting agent placed in a space between the container and the casing, wherein the oxygen absorber does not affect the pH or the bicarbonate ion concentration of the solution.

2. The reservoir assembly according to claim 1, wherein the oxygen absorber mainly contains a crosslinked polymer having carbon-carbon unsaturated bonds.

3. The reservoir assembly according to claim 1 or 2, wherein the space between the container and the casing is filled with a gas atmosphere having a carbon dioxide concentration in equilibrium with the pH of the solution, so that changes in the pH of the solution and in the amount of carbon dioxide in the container are minimized during packaging of the container.

4. The reservoir assembly according to any of claims 1 to 3, wherein the gas-impermeable casing has an oxygen permeability of 0.7 mL/m²/day/atom or less.

5. The reservoir assembly according to any of claims 1 to 4, wherein the solution has a pH in the range of 6.8 to 7.8.

6. The reservoir assembly according to any of claims 1 to 5, wherein the gas atmosphere filling the space between the container and the casing contains carbon dioxide at a concentration in the range of 1 to 19 v/v% and oxygen at a concentration of 0.1% or less.
